## Europäisches Patentamt
## European Patent Office
## Office européen des brevets

(11) Numéro de publication : **0 027 767**
**B1**

(12) # FASCICULE DE BREVET EUROPÉEN

(45) Date de publication du fascicule du brevet :
**28.12.83**

(21) Numéro de dépôt : **80401485.0**

(22) Date de dépôt : **17.10.80**

(51) Int. Cl.³ : **G 01 N  1/00**, G 01 N 33/20,
G 01 M  3/20, G 21 C 17/02

(54) **Dispositif de détection d'une substance dont on cherche à déterminer la présence dans une ou plusieurs chambres d'un ensemble de chambres de mesure maintenues sous ultra-vide et application à la détection des fuites.**

(30) Priorité : **18.10.79 FR 7925902**

(43) Date de publication de la demande :
**29.04.81 Bulletin 81/17**

(45) Mention de la délivrance du brevet :
**28.12.83 Bulletin 83/52**

(84) Etats contractants désignés :
**BE DE GB IT NL**

(56) Documents cités :
**DE-C-  562 575
FR-A- 2 373 049
US-A- 3 731 523
US-A- 3 801 440**

(73) Titulaire : **NOVATOME
20 Avenue Edouard Herriot
F-92350 Le Plessis Robinson (FR)**

(72) Inventeur : **Le Baud, Patrice
60, rue Marie Fichet
F-92140 Clamart (FR)**

(74) Mandataire : **Bouget, Lucien et al
CREUSOT-LOIRE 15 rue Pasquier
F-75383 Paris Cedex 08 (FR)**

Jouve, 18, rue St-Denis, 75001 Paris, France

Dispositif de détection d'une substance dont on cherche à déterminer la présence dans une ou plusieurs chambres d'un ensemble de chambres de mesure maintenues sous ultra-vide et application à la détection des fuites

L'invention concerne un dispositif de détection d'une substance dont on cherche à déterminer la présence dans une ou plusieurs chambres d'un ensemble de chambres de mesure maintenues sous ultra-vide, par mesure de pression dans chacune des chambres de mesure successivement.

En particulier, l'invention concerne un dispositif permettant la détection d'hydrogène dans un métal liquide, en divers points de prélèvement, dans un circuit comportant un échangeur de chaleur où le métal liquide constitue l'un des fluides d'échange et où l'autre fluide d'échange renferme de l'hydrogène.

Dans les centrales nucléaires à neutrons rapides, on utilise des échangeurs de chaleur où les fluides d'échange sont constitués par du sodium liquide qui constitue le fluide caloporteur primaire et secondaire du réacteur et par de l'eau qui fournit par vaporisation le fluide moteur entraînant la turbine.

Il est extrêmement important de surveiller l'intégrité des surfaces d'échange séparant les fluides dans ces échangeurs, pour détecter la présence ou l'apparition de petites fissures dans ces surfaces d'échange afin d'éviter l'apparition d'accidents majeurs pouvant entraîner une destruction d'une partie ou de la totalité de l'échangeur.

Il est donc extrêmement important de détecter des fuites possibles au niveau du générateur de vapeur où s'effectuent les échanges calorifiques entre le sodium liquide et l'eau, pour la vaporisation de celle-ci. Généralement le générateur de vapeur est constitué par des tubes de petit diamètre à l'intérieur desquels passe l'eau pour sa vaporisation, les tubes étant disposés à l'intérieur de l'enceinte du générateur de vapeur dans laquelle s'écoule du sodium liquide venant en contact avec leur surface externe. Bien que de nombreuses précautions soient prises pendant la fabrication et le contrôle des tubes utilisés dans les générateurs de vapeur, ces tubes peuvent néanmoins présenter après un temps plus ou moins long de fonctionnement de la centrale de petites fissures génératrices de fuites qui risquent de s'ouvrir plus largement pendant le fonctionnement ultérieur du générateur pour aboutir à un phénomène d'auto-évolution extrêmement rapide de la fuite pouvant conduire à la ruine d'une partie importante du générateur de vapeur.

Il est donc important de déceler les fuites éventuelles lorsqu'elles sont encore très faibles et éloignées de l'état d'auto-évolution.

Une fuite dans un tube du générateur de vapeur se traduit, compte tenu de la pression importante du côté de l'eau ou de la vapeur d'eau par rapport à celle du sodium liquide, par une contamination liquide par l'eau ou la vapeur d'eau.

On a proposé divers procédés pour détecter de façon très sensible des traces d'eau dans le sodium liquide des générateurs de vapeur et en particulier on utilise couramment une mesure de la concentration d'hydrogène dans le sodium liquide en divers points du circuit sodium contenant le générateur de vapeur.

Pour mesurer cette concentration d'hydrogène, on met en contact avec l'une des faces d'une paroi de nickel de sodium liquide prélevé au point du générateur de vapeur où l'on désire effectuer la mesure de la concentration d'hydrogène, l'autre face de la paroi de nickel étant soumise à une très faible pression. On mesure alors le flux de l'hydrogène diffusant à travers la paroi de nickel dans le milieu sous très faible pression et l'on en déduit la concentration d'hydrogène dans le sodium liquide prélevé dans le générateur de vapeur.

On a par exemple décrit dans le brevet US-A-3 731 523, un dispositif pour la détection d'hydrogène dans du sodium liquide, par mesure de la pression due à l'hydrogène diffusant dans une chambre maintenue sous très faible pression par une installation de pompage et séparée du sodium liquide par une paroi perméable à l'hydrogène à la température du sodium liquide. Un tel dispositif comporte, outre l'installation de pompage permettant d'établir un vide poussé (ultra-vide) dans la chambre, un moyen de mesure de la pression dans la chambre qui est fonction de la concentration de l'hydrogène dans le sodium liquide.

Les générateurs de vapeur des centrales nucléaires à neutrons rapides comportent généralement des systèmes de fixation des tubes contenant l'eau ou la vapeur qui cloisonnent le circuit sodium en plusieurs secteurs où les écoulements sont pratiquement indépendants les uns des autres sur la plus grande partie de la hauteur du générateur de vapeur.

A l'intérieur de chacun de ces secteurs le sodium liquide qui s'écoule verticalement présente, en cas de fuite dans l'un des secteurs, une concentration en hydrogène très supérieure à la concentration en hydrogène du sodium dans les autres parties du générateur de vapeur.

Jusqu'ici on ne connaissait pas de dispositif permettant d'effectuer rapidement et successivement des mesures de concentration d'une substance telle que l'hydrogène sur des prélèvements de sodium liquide provenant de divers points d'une installation où circule ce sodium liquide.

On connaît cependant (brevet FR-A-2 373 049) un dispositif de détection d'hydrogène dans du sodium liquide par mesure de pression comportant deux chambres de mesure sous ultra-vide séparées du sodium liquide chacune par une paroi perméable à l'hydrogène et reliées à une installation de pompage unique. Des moyens de mesure différents permettent de déterminer la pression d'hydrogène en régime statique dans l'une des chambres et en régime dynamique dans l'autre chambre.

Un tel dispositif ne permet pas de déterminer des valeurs comparables de la pression de l'hydrogène dans des chambres de mesure en communication, par l'intermédiaire de parois perméables à l'hydrogène, avec divers points d'un circuit de sodium liquide.

Le but de l'invention est donc de proposer un dispositif de détection d'une substance dont on cherche à déterminer la présence dans une ou plusieurs chambres d'un ensemble de chambres de mesure maintenues sous ultra-vide par une installation de pompage unique et reliées chacune par l'intermédiaire d'un organe permettant un passage limité et sélectif de la substance, à un point d'un circuit ou d'une installation renfermant un fluide constituant la substance ou contenant la substance, par mesure de pression dans chacune des chambres successivement, dispositif qui permette des mesures successives de pression en un temps très court sur l'ensemble des chambres de mesure et de façon à ce que ces mesures soient représentatives de la présence d'une certaine quantité même très faible de la substance, en évitant les effets perturbateurs qui peuvent être dus aux moyens de mesure de pression et au dispositif de pompage sous vide.

Dans ce but, les chambres de mesure sont reliées à l'installation de pompage unique par l'intermédiaire d'éléments à faible conductance, c'est-à-dire offrant une grande résistance au passage des molécules et sont reliées d'autre part à un moyen de mesure de pression commun à l'ensemble des chambres par l'intermédiaire d'un sélecteur permettant de mettre en communication successivement chacune des chambres avec le moyen de mesure de pression.

Afin de bien faire comprendre l'invention, on va maintenant décrire à titre d'exemple non limitatif en se reportant aux figures jointes en annexe un mode de réalisation du dispositif de mesure suivant l'invention, dans le cas où ce dispositif est utilisé pour mesurer la teneur en hydrogène en divers points de prélèvement du circuit sodium d'un échangeur de chaleur d'un réacteur à neutrons rapides.

La figure 1 représente, dans une vue en coupe par un plan de symétrie, l'ensemble du dispositif de mesure.

La figure 2 représente, dans une vue en coupe par un plan de symétrie, le sélecteur du circuit sous très faible pression résiduelle.

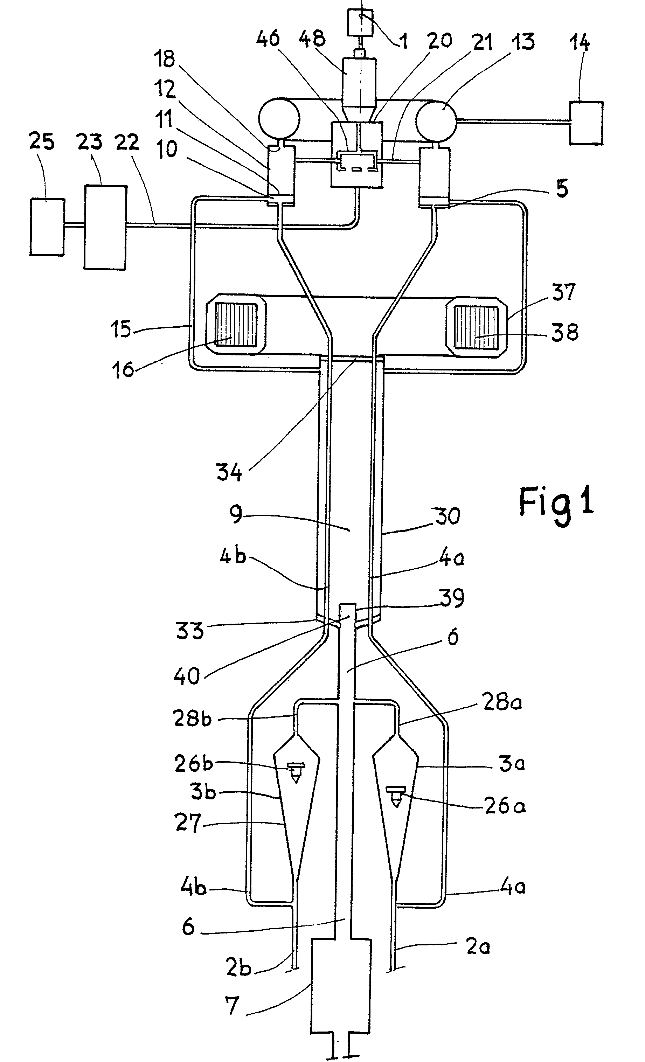

Sur la figure 1 est représenté, en coupe par un plan de symétrie, un dispositif de mesure utilisable pour déterminer la teneur en hydrogène du sodium liquide dans les huit secteurs aussi appelés octants d'un générateur de vapeur d'un réacteur nucléaire à neutrons rapides.

Ce dispositif de mesure comporte huit circuits de prélèvement et de séparation d'hydrogène identiques disposés avec un décalage angulaire de 45° autour de l'axe 1 de symétrie du dispositif, si bien que deux circuits seulement sont visibles sur la coupe de la figure 1.

On utilisera les mêmes repères pour désigner les éléments correspondants des deux circuits, l'ensemble des repères relatif à l'un des circuits étant affecté de l'indice a et l'ensemble des repères relatif aux éléments de l'autre circuit étant affecté de l'indice b.

Chacun des circuits comporte une conduite de prélèvement 2 reliée à l'une de ses extrémités au point de prélèvement correspondant sur le générateur de vapeur et à son autre extrémité à la partie inférieure d'un régulateur de débit 3.

A la partie basse du régulateur de débit 3 est reliée la conduite d'arrivée du métal liquide 4 recevant le métal liquide à débit constant alors que la partie supérieure du régulateur de débit 3 renvoie une partie du métal liquide dans la conduite de retour 6 dans laquelle le sodium liquide est mis en circulation par une pompe 7 qui permet le retour de ce métal liquide dans le générateur de vapeur.

L'ensemble des huit régulateurs correspondant à chacun des circuits de prélèvement sont en communication avec la conduite 6 dans laquelle se rejoignent les différents courants de sodium liquide après traversée des régulateurs 3.

La pompe unique 7 permet de mettre en circulation le sodium liquide dans l'ensemble des circuits de mesure.

Les huit conduites d'arrivée de métal liquide 4a, 4b, ..., 4h traversent l'échangeur économiseur 9 sur toute sa longueur, ce qui permet un réchauffage du sodium par échange avec le sodium de retour arrivant à contre-courant.

A leur sortie de l'échangeur économiseur 9, les conduites d'arrivée de sodium liquide 4 pénètrent dans les chambres 10 des dispositifs de séparation d'hydrogène (5) dans chacune desquelles le sodium liquide réchauffé dans l'échangeur économiseur vient en contact avec la paroi de nickel 11 sur sa face opposée à la chambre 12 elle-même reliée à une enceinte 13 dans laquelle une pompe ionique unique 14 établit une très faible pression de l'ordre de $10^{-9}$ Torr.

Le sodium liquide après être venu en contact avec la membrane de nickel 11 ressort de la chambre 10 du dispositif de séparation d'hydrogène par la conduite de retour 15 dans laquelle le sodium liquide se réchauffe grâce à un dispositif de chauffage par induction 16 avant de retourner dans le corps de l'échangeur 9 où le sodium réchauffé vient en contact avec les conduites 4 d'arrivée du sodium liquide au dispositif de séparation d'hydrogène.

Après traversée de l'échangeur économiseur 9, le sodium s'écoule dans le collecteur de retour 6, l'ensemble de la circulation du sodium étant assuré par la pompe.

Il se produit donc une circulation continue du sodium prélevé dans chacun des huit octants du générateur de vapeur, dans tout le circuit sodium en particulier entre les points de prélèvement sur l'échangeur de chaleur et les membranes de nickel 11.

Les chambres 12 des dispositifs de séparation d'hydrogène sont mises en communication permanente avec l'enceinte 13 de forme torique maintenue sous ultra-vide grâce à la pompe 14, par l'intermédiaire de diaphragmes réglables 18 qui permettent d'établir une chute de pression entre la chambre 12 et l'enceinte 13, la pression dans la chambre 12 étant de l'ordre de $10^{-7}$ à $10^{-8}$ Torr alors que la pression est de l'ordre de $10^{-9}$ Torr dans l'enceinte 13, le réglage des diaphragmes permettant lors de l'étalonnage de l'appareil d'équilibrer parfaitement les pressions d'hydrogène dans les chambres 12.

Chacune des chambres 12 sous ultra-vide est reliée d'autre part au sélecteur 20 par une conduite latérale 21.

Le sélecteur 20 est en rotation continue et permet de mettre en communication successivement chacun des conduits latéraux 21 avec un conduit 22 lui-même relié au spectromètre de masse 23 permettant la mesure de la pression de l'hydrogène dans chacune des chambres 12 successivement.

Les signaux émis par le spectromètre de masse sont traités dans une unité de calcul 25 qui permet d'effectuer la comparaison entre les mesures de pression d'hydrogène dans chacune des chambres 12.

Le dispositif régulateur de débit associé à chacun des circuits est constitué par une conduite verticale de forme conique 27 à l'intérieur de laquelle est disposé un flotteur 26 qui trouve sa position d'équilibre dans le conduit conique 27 en fonction du débit vertical ascendant de sodium liquide venant par la conduite de prélèvement 2.

La partie supérieure de la conduite conique 27 est reliée par la conduite 28 à la conduite de retour 6 à l'aval du point où cette conduite 6 est reliée au corps de l'échangeur économiseur 9.

La conduite d'arrivée de sodium liquide 4 est placée en dérivation à l'entrée du régulateur 3 c'est-à-dire à la base de la conduite conique 27.

Chacun des circuits de sodium liquide comporte un régulateur 3 recevant à sa base le sodium liquide de la conduite de prélèvement et relié à sa partie supérieure à une conduite 28 alors que le conduit d'arrivée de sodium liquide dans le dispositif de séparation est monté en dérivation à la base du régulateur.

Deux régulateurs disposés à 180° ont été représentés à la figure 1.

Si des différences de débit apparaissent pour les flux de sodium liquide arrivant par les différentes conduites de prélèvement 2, les flotteurs 26 vont se placer dans la conduite 27 en des positions verticales différentes.

Par exemple, sur la figure 1 les flotteurs 26a et 26b sont dans des positions correspondant à des débits de sodium différents dans les conduites 2a et 2b. L'ensemble des éléments du régulateur et en particulier les flotteurs 26 disposés dans chacune des conduites 27 des différents régulateurs de débit sont réalisés de façon que la perte de charge soit identique pour chacun des circuits de prélèvement.

La perte de charge entre l'entrée du régulateur d'où partent la conduite d'arrivée 4 et la sortie du régulateur dans la conduite de retour 6 est donc identique pour tous les circuits.

Il en résulte donc que les débits dans les différentes conduites d'arrivée de sodium liquide 4 sont identiques, les différences de débit dans les conduites de prélèvement (2) étant absorbées par des différences de débit dans les régulateurs provoquées par les différences de position des flotteurs 26.

Les débits de sodium liquide dans les conduites d'arrivée 4 et dans les différentes chambres 10 des dispositifs de séparation sont donc tous identiques pour les différents circuits de prélèvement et constants au cours du temps, quels que soient les débits dans les conduites de prélèvement.

L'échangeur économiseur 9 est constitué par une enveloppe cylindrique 30 fermée à ses extrémités par des plaques 33 et 34 traversées par les tubes d'arrivée 4 du sodium liquide disposés avec une égale répartition angulaire autour de l'axe de l'échangeur économiseur.

Après la traversée de l'échangeur économiseur 9, chacun des tubes d'arrivée de sodium liquide 4 est relié à la chambre interne 10 du dispositif de séparation d'hydrogène 5 comportant les chambres 10 et 12 et une membrane de nickel 11 séparant ces deux chambres.

La conduite 15 de retour du sodium liquide débouche dans le corps 30 de l'échangeur économiseur à proximité de la plaque 34, chacune des conduites de retour 15 constituant une boucle entourant le dispositif de réchauffage 16 constitué par un circuit magnétique 38 entouré par un bobinage 37 alimenté en courant alternatif. La fermeture de cette boucle est assurée par une portion de la conduite d'arrivée 4 correspondante.

Le sodium liquide circulant dans la conduite de retour 15 entourant le dispositif 16 est chauffé par induction avant son retour dans l'échangeur économiseur, ce qui permet d'introduire dans le corps de cet échangeur du sodium effectuant le réchauffage par circulation à contre-courant du sodium circulant dans les tubes 4.

Le sodium réintroduit par les conduites de retour 15 dans le corps de l'échangeur traverse tout le corps de l'échangeur et s'écoule dans un tube 40 traversant la plaque 33 et communiquant avec le tube de retour du sodium liquide 6.

L'ensemble de la conduite de retour 15 du corps de l'échangeur économiseur, du tube 40 et du tube 6 constitue le conduit de retour du sodium liquide où celui-ci est mis en circulation par la pompe 7.

On voit donc que l'ensemble des flux de sodium liquide réchauffé dans les différentes conduites de retour 15 se mélange dans le corps de l'échangeur économiseur si bien que les tubes 4 sont en contact avec un fluide d'échange à température homogène dans une section donnée. De cette façon, le sodium arrivant par les tubes 4 est porté à la même température pour chacun des circuits avant son entrée dans les chambres 10.

La diffusion de l'hydrogène se produit donc dans les mêmes conditions de température pour chacun des circuits.

L'apparition d'une fuite d'eau ou de vapeur dans une partie de l'échangeur de chaleur provoque une augmentation de la concentration d'hydrogène dans un des circuits de prélèvement, la diffusion de cet hydrogène à travers la paroi 11 correspondante augmente la pression dans la chambre 12 du séparateur 5 mais grâce au diaphragme 18 qui est un élément à faible conductance, cette élévation de pression interfère peu avec la pression résiduelle dans les chambres 12 des autres dispositifs de séparation d'hydrogène.

De cette façon, la pression résiduelle dans l'une des chambres 12 peut varier par exemple entre $10^{-7}$ et $10^{-8}$ Torr sans perturber notablement la pression dans les autres chambres 12.

De cette façon, il est possible d'utiliser un circuit sous ultra-vide comportant une enceinte et une pompe ionique commune à l'ensemble des dispositifs de séparation d'hydrogène sans qu'il y ait d'interférence sensible entre les différentes mesures.

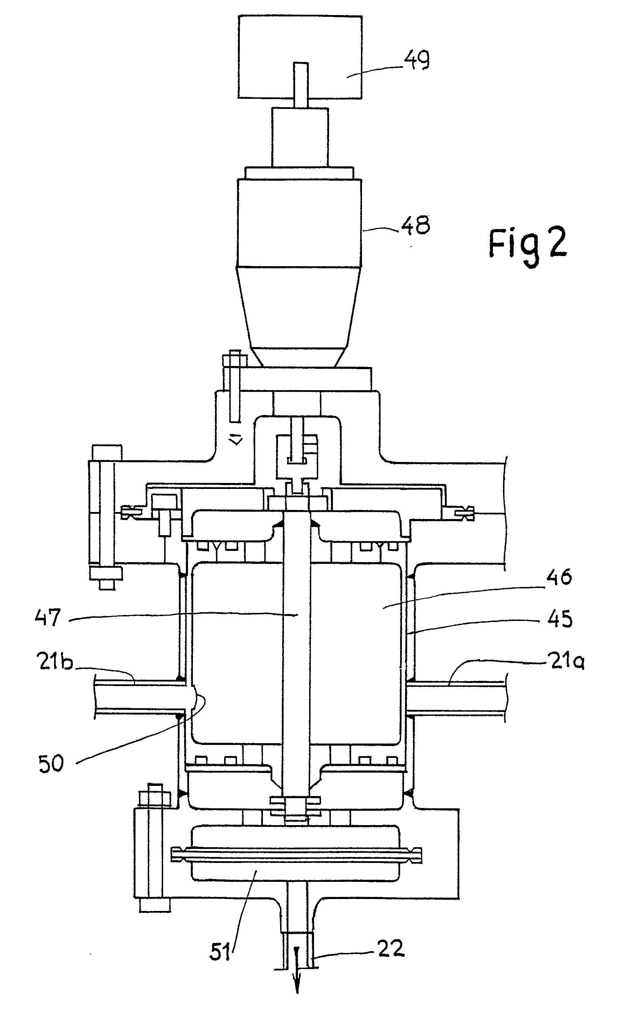

En se référant à la figure 2, on voit une coupe par un plan de symétrie du dispositif sélecteur 20 permettant de réunir successivement chacune des chambres 12 des dispositifs de séparation d'hydrogène au circuit de mesure constitué par la conduite 22 et le spectromètre 23.

Ce sélecteur comporte un corps 45 cylindrique renfermant un cylindre sélecteur 46 mis en rotation par l'intermédiaire d'un arbre 47 relié par une traversée étanche 48 à un moteur pas à pas 49 effectuant huit pas par tour, les différentes positions du cylindre sélecteur correspondant à la mise en communication des conduites 21 des différentes chambres 12 avec la conduite 22 sur laquelle est disposé le spectromètre de masse.

A chacun des pas du moteur 49 est effectuée ainsi la mesure de la pression d'hydrogène dans la chambre 12 du dispositif séparateur dont la conduite 21 se trouve en face de l'ouverture 50 du cylindre sélecteur constitué par un cylindre creux en communication avec une cavité 51 dans laquelle débouche la conduite 22 reliée au spectromètre de masse.

Le cylindre sélecteur comporte une seule ouverture 50 qui vient successivement se mettre en position devant les conduites 21, les autres conduites 21 étant alors masquées par la surface latérale du cylindre sélecteur 46.

On comprend que le sélecteur peut réaliser des pas successifs à grande vitesse et qu'ainsi le balayage des huit voies de mesure correspondant à l'ensemble du générateur de vapeur peut être effectué très rapidement.

A chaque pas, le spectromètre de masse réalise une mesure de la pression d'hydrogène dans la chambre 12 correspondante qui est directement reliée à la concentration d'hydrogène dans le sodium liquide du prélèvement correspondant.

Les valeurs de mesure stockées au niveau de l'unité de calcul 25 au cours d'un ou plusieurs tours complets du sélecteur sont traitées de façon globale et comparative.

Par exemple, la moyenne des différentes valeurs de pression d'hydrogène relevées peut être calculée, pour un tour, et comparée à chacune de ces valeurs ou bien, la variation de pression sur une voie peut être comparée aux variations de pression sur les autres voies.

En se fixant des bornes prédéterminées pour les écarts possibles entre les valeurs mesurées et la valeur moyenne ou pour les écarts de variation entre voies on peut déterminer la présence d'une fuite sur l'une des huit parties du générateur de vapeur, ce qui permet d'assurer la surveillance du générateur de vapeur aussi bien pour ses états stables que pour les phases transitoires.

On voit que les principaux avantages du dispositif selon l'invention sont de permettre une succession rapide de mesures sur les différents circuits de prélèvement, avec un métal liquide s'écoulant à débit constant et présentant une température constante au niveau des membranes dans les différents circuits, tout en permettant des mesures comparatives tout à fait significatives permettant de déceler des fuites très faibles. Le fait d'utiliser une seule installation de pompage en vide réunie aux chambres de mesure par des éléments à faible conductance et un seul moyen de mesure permet d'éviter des effets perturbateurs dus aux variations de caractéristiques des appareils.

Mais l'invention ne se limite pas au mode de réalisation qui vient d'être décrit ; elle en comporte au contraire toutes les variantes qu'autorise la portée des revendications.

Dans le cas où l'on utilise le dispositif pour la mesure d'un paramètre relatif à une substance telle que de l'hydrogène en mélange avec un métal liquide, comme il a été décrit dans l'exemple de réalisation, on peut apporter certaines modifications au dispositif décrit dans l'exemple.

C'est ainsi qu'on peut imaginer des dispositifs de régulation de débit du métal liquide d'un type différent de celui qui a été décrit, que l'échangeur économiseur peut être d'un type différent d'un échangeur à contre-courant, que le réchauffage du sodium peut être d'un type différent d'un réchauffeur par induction dans les conduites de retour avec mélange dans le corps de l'échangeur.

Le moyen de mesure peut également être différent d'un spectromètre de masse et le paramètre mesuré peut être aussi bien la pression partielle de la substance dans la chambre de mesure que la pression totale dans cette chambre de mesure puisque cette pression totale dépend également de la présence de la substance diffusant éventuellement dans la chambre. On peut par exemple utiliser une jauge d'un type convenable pour la mesure de très faibles pressions.

A la place d'une membrane de nickel, on peut utiliser toute autre membrane à travers laquelle l'hydrogène diffuse, lorsque cette membrane est en contact d'un côté avec le métal liquide et de l'autre avec une chambre sous pression résiduelle très faible.

Il est possible également d'utiliser le dispositif de mesure suivant l'invention en dehors du domaine des générateurs de vapeur des réacteurs nucléaires à neutrons rapides et plus généralement en dehors du domaine du contrôle des installations comprenant un échangeur de chaleur ayant pour fluides d'échange un métal liquide et un fluide contenant de l'hydrogène. On peut ainsi utiliser le dispositif suivant l'invention pour contrôler l'étanchéité en divers points d'une installation ou d'un circuit renfermant un fluide. Il suffit pour cela de relier chacune des chambres de mesure du dispositif à une zone voisine de chaque point particulier du circuit à contrôler, par l'intermédiaire d'un organe permettant un passage limité de la substance dans la chambre de mesure, en cas de fuite au point considéré dans l'atmosphère ambiante.

Il est en effet nécessaire de limiter le passage de la substance vers la chambre sous vide pour pouvoir effectuer les mesures sous pression très faible.

On peut utiliser comme organe limitant le passage de la substance vers la chambre sous vide une paroi diffusante métallique ou non métallique ou encore un tube capillaire ou un ensemble de tubes capillaires.

La substance dont on mesure un paramètre, par exemple la pression, dans la chambre de mesure peut constituer à elle seule le fluide contenu dans le circuit ou l'installation à contrôler. Cette substance peut être également un fluide en mélange avec le fluide remplissant l'installation.

La substance sur laquelle on effectue la mesure peut se trouver dans le fluide à titre accidentel ou au contraire être introduite à titre de traceur, en vue de contrôler des fuites éventuelles.

Dans le cas de deux fluides séparés par une paroi d'échange, le contrôle de l'intégrité de cette paroi peut être fait en reliant des zones voisines de divers points de la paroi à des chambres de mesures d'un dispositif selon l'invention par l'intermédiaire d'une paroi diffusante par exemple. La substance à contrôler dans ce cas peut être l'un des fluides d'échange qui a pu être introduit accidentellement dans l'autre fluide. Ce peut être aussi un fluide introduit comme traceur dans l'un des fluides d'échange, les prélèvements étant effectués sur l'autre fluide d'échange.

Enfin, le dispositif peut être utilisé pour le contrôle d'enceintes constituant les chambres de mesure elles-mêmes, si une substance du milieu extérieur est susceptible de pénétrer dans ces enceintes, en cas de défaut d'étanchéité.

Le sélecteur en ultra-vide permettant d'effectuer les mesures sur chaque chambre successivement peut avoir une forme et une structure différentes de celles qui ont été décrites.

Dans le cas où ce sélecteur est constitué par un cylindre monté rotatif dans une enceinte cylindrique étanche, ce cylindre peut être un cylindre creux comme dans l'exemple de réalisation qui a été décrit ou un cylindre plein comportant des perçages usinés à sa partie interne dont l'un à disposition radiale peut venir dans l'alignement des conduits reliant l'enceinte du sélecteur aux chambres sous vide et communique avec au moins un autre perçage permettant de le relier à une partie de l'enceinte du sélecteur en communication avec le moyen de mesure.

Le dispositif suivant l'invention s'applique dans tous les cas où l'on désire mesurer un paramètre relatif à une substance présente en très faible quantité dans une ou plusieurs chambres parmi un ensemble de chambres sous vide, la comparaison des mesures effectuées successivement sur les différentes chambres permettant une détection soit qualitative, soit quantitative de la substance, avec une très grande sensibilité.

## Revendications

1. Dispositif de détection d'une substance dont on cherche à déterminer la présence dans une ou plusieurs chambres (12) d'un ensemble de chambres de mesure maintenues sous ultra-vide par une installation de pompage unique (14) et reliées chacune par l'intermédiaire d'un organe (11) permettant un passage limité et sélectif de la substance à un point d'un circuit ou d'une installation renfermant un fluide constituant la substance ou contenant la substance, par mesure de pression dans chacune des chambres de mesure (12) successivement, caractérisé par le fait que les chambres de mesure (12) sont reliées à l'installation de pompage unique (14), par l'intermédiaire d'éléments (18) à faible conductance, c'est-à-dire offrant une grande résistance au passage des molécules et sont reliées d'autre part à un moyen de mesure de pression (23) commun à l'ensemble des chambres, par l'intermédiaire d'un sélecteur (20) permettant de mettre en communication successivement chacune des chambres (12) avec le moyen de mesure de pression (23).

2. Dispositif de détection suivant la revendication 1, caractérisé par le fait que l'organe (11) offrant un passage limité à la substance est une paroi permettant la diffusion de la substance.

3. Dispositif de détection suivant la revendication 1, caractérisé par le fait que l'organe (11) offrant un passage limité à la substance est constitué par au moins un tube capillaire.

4. Dispositif de détection suivant la revendication 2, dans le cas où la substance sur laquelle on effectue la mesure est de l'hydrogène contenu dans un métal liquide circulant dans une installation comportant un échangeur de chaleur où le métal liquide constitue l'un des fluides d'échange et où l'autre fluide d'échange renferme de l'hydrogène, caractérisé par le fait qu'il comporte un ensemble de circuits de prélèvement de métal liquide et de séparation d'hydrogène, chaque circuit étant associé à un point de prélèvement disposé à un endroit particulier de l'installation et comprenant :

— un conduit d'arrivée (4) du métal liquide recevant le métal liquide à débit constant et traversant un échangeur de chaleur économiseur (9) commun à l'ensemble des circuits,

— et un dispositif de séparation d'hydrogène comportant une première chambre (10) reliée d'une part au conduit d'arrivée (4) de métal liquide et d'autre part à un conduit de retour (15) de ce métal à l'échangeur économiseur (9) et une seconde chambre constituant la chambre de mesure (12) sous très faible pression séparée de la première chambre par une paroi métallique (11).

5. Dispositif de détection suivant la revendication 4, caractérisé par le fait que les conduites d'arrivée (4) sont placées en dérivation chacune sur une conduite de prélèvement (2) sur laquelle est placé un régulateur de débit du métal liquide (27), en aval de l'embranchement de la conduite d'arrivée (4).

6. Dispositif de détection selon l'une quelconque des revendications 4 et 5, caractérisé par le fait que l'échangeur économiseur (9) est constitué par un corps creux (30) enveloppant les conduits d'arrivée de métal liquide (4) et constituant le circuit de retour du métal liquide, en aval des conduits de retour (15) des différents circuits de prélèvement qui sont tous en communication à leur extrémité avec le corps de l'échangeur économiseur, un dispositif de réchauffage (16) permettant d'élever la température du métal liquide en retour à l'intérieur des conduits (15) des circuits de prélèvement, en amont de l'échangeur économiseur (9), les conduits d'arrivée (4) étant ainsi réchauffés par le métal liquide provenant des différents circuits de prélèvement et mélangé au niveau de l'échangeur économiseur.

7. Dispositif suivant la revendication 6, caractérisé par le fait que le dispositif de réchauffage (16) du métal dans les conduits de retour (15) des circuits de prélèvement est constitué par un circuit magnétique (38) unique sur lequel est enroulé un bobinage (37) pour la création de l'induction assurant le chauffage du métal liquide dans les conduits (15).

8. Dispositif de mesure suivant la revendication 5, caractérisé par le fait que le régulateur de débit du métal liquide est constitué, pour chacun des circuits, par une portion de canalisation conique (27) disposée avec son axe vertical, alimentée à sa base par le conduit de prélèvement (2) et reliée à sa partie supérieure à la conduite de retour du métal liquide (6), renfermant un flotteur (26) obstruant plus ou moins la canalisation conique suivant sa position verticale dans ce conduit, le conduit d'arrivée (4) du métal liquide étant disposé en dérivation à la base du conduit conique (27) et chacun des dispositifs régulateurs de débit (27) associés à chacun des circuits de prélèvement créant une perte de charge identique dans chaque circuit de prélèvement.

9. Dispositif de détection suivant l'une quelconque des revendications 1 à 8, caractérisé par le fait que le sélecteur (20) est constitué par une enceinte cylindrique (45) étanche à l'intérieur de laquelle est disposé un cylindre sélecteur (46) coaxial à l'enceinte (45) et monté rotatif autour de son axe dans cette enceinte comportant une ouverture latérale (50) permettant de mettre en communication une partie (51) de l'enceinte du sélecteur reliée au moyen de mesure de pression (23) successivement avec des conduits (21) reliant l'enceinte (45) du sélecteur à chacune des chambres sous vide (12), ces circuits étant disposés radialement sur l'enceinte (45) en des emplacements tels que la mise en rotation du cylindre sélecteur (46) par un moyen moteur (49) qui lui est relié mettent en coïncidence l'ouverture (50) dans la paroi latérale du cylindre sélecteur successivement avec les conduits (21) disposés radialement sur l'enceinte, pour assurer la mise en communication successive des différentes chambres sous vide (12) avec le système de mesure de pression (23).

10. Dispositif de détection suivant l'une quelconque des revendications 1 à 9, caractérisé par le fait que les éléments à faible conductance (18) ont une conductance réglable indépendamment les uns des autres.

11. Dispositif de détection suivant l'une quelconque des revendications 1 à 10, caractérisé par le fait que le moyen de mesure de pression (23) est un dispositif de mesure de la pression totale dans la chambre de mesure.

12. Dispositif suivant l'une quelconque des revendications 1 à 11, caractérisé par le fait que le moyen de mesure de pression (23) est relié à une unité de calcul (25) pour le traitement des signaux émis par ce moyen de mesure.

13. Application d'un dispositif de mesure suivant l'une quelconque des revendications 1, 2 et 3, au contrôle de fuites en divers points d'un circuit ou d'une installation renfermant au moins un fluide, caractérisée par le fait qu'on effectue des prélèvements dans des zones voisines de divers points du circuit ou de l'installation pour déceler des traces de fluide dans l'atmosphère entourant le circuit ou l'installation.

14. Application d'un dispositif de mesure suivant l'une quelconque des revendications 1, 2 et 3, au contrôle de l'étanchéité d'une paroi d'échange séparant deux fluides dans une installation telle qu'un échangeur de chaleur caractérisée par le fait qu'on introduit une substance à titre de traceur dans l'un des fluides, ou premier fluide, et qu'on effectue des prélèvements de second fluide en divers points de l'installation pour déceler dans ces prélèvements la présence éventuelle du traceur constituant la substance sur laquelle a lieu la mesure.

**Claims**

1. Device for detecting a substance the presence of which is to be determined in one or more chambers (12) of an assembly of measuring chambers which are maintained under an ultrahigh vacuum by a single pumping system (14) and which are each connected, via a means (11) allowing limited and selective passage of the substance, to a point of a circuit or of an installation containing a fluid constituting the substance or containing the substance, by measuring the pressure in each of the measuring chambers (12) in succession, characterised in that the measuring chambers (12) are connected to the single pumping system (14) by means of low-conductivity elements (18), that is to say elements presenting high resistance to the passage of the molecules, and are connected on the other hand to a pressure-measuring means (23), common to all the chambers, by means of a selector (20) allowing each of the chambers (12) to be put in communication with the pressure-measuring means (23) in succession.

2. Detection device according to Claim 1, characterised in that the means (11) presenting a limited passage to the substance is a wall which allows the diffusion of the substance.

3. Detection device according to Claim 1, characterised in that the means (11) presenting a limited passage to the substance consists of at least one capillary tube.

4. Detection device according to Claim 2, where the substance which is measured is hydrogen contained in a liquid metal circulating in a system incorporating a heat exchanger in which the liquid metal constitutes one of the exchange fluids and in which the other exchange fluid contains hydrogen, characterised in that it incorporates an assembly of liquid-metal sampling and hydrogen-separation circuits, each circuit being associated with a sampling point located in a particular place in the system and comprising a liquid-metal inflow pipe (4) which receives the liquid metal at a constant rate and which passes through an energy-saving heat exchanger (9) common to all the circuits, and a hydrogen-separation device comprising a first chamber (10), connected on the one hand to the liquid-metal inflow pipe (4) and on the other hand to a return pipe (15) for returning this metal to the energy-saving exchanger (9), and a second chamber forming the measuring chamber (12) under very low pressure which is separated from the first chamber by a metal wall (11).

5. Detection device according to Claim 4, characterised in that the inflow pipes (4) are each branched on a sampling pipe (2) on which a liquid-metal flow regulator (27) is located downstream of the branching of the inflow pipe (4).

6. Detection device according to either one of Claims 4 and 5, characterised in that the energy-saving exchanger (9) consists of a hollow body (30) which encases the liquid-metal inflow pipes (4) and constitutes the liquid-metal return circuit, downstream of the return pipes (15) of the various sampling circuits which all communicate at their end with the body of the energy-saving exchanger, a heating device (16) making it possible to raise the temperature of the liquid metal returning within the pipes (15) of the sampling circuits, upstream of the energy-saving exchanger (9), the inflow pipes (4) thereby being heated by the liquid metal coming from the various sampling circuits and mixed at the level of the energy-saving exchanger.

7. Device according to Claim 6, characterised in that the heating device (16) for the metal in the return pipes (15) of the sampling circuits consists of a single magnetic circuit (38) on which a coil (37) is wound for generating the induction which ensures the heating of the liquid metal in the pipes (15).

8. Measuring device according to Claim 5, characterised in that the liquid-metal flow regulator consists, for each of the circuits, of a conical pipeline portion (27) which has a vertical axis and is supplied at its base via the sampling pipe (2) and which is connected in its upper part to the liquid-metal return pipe (6) and contains a float (26) which obstructs the conical pipeline to a greater or lesser extent according to its vertical position in this pipe, the liquid-metal inflow pipe (4) being branched at the base of the conical pipe (27), and each of the flow regulator devices (27) associated with each of the sampling circuits generating an identical charge loss in each sampling circuit.

9. Detection device according to any one of Claims 1 to 8, characterised in that the selector (20) consists of a leak-proof cylindrical enclosure (45) within which a selector cylinder (46) coaxial with the enclosure (45) is located and mounted rotatably about its axis in this enclosure which has a lateral orifice (50) making it possible to put a portion (51) of the enclosure of the selector connected to the pressure-measuring means (23) in communication successively with pipes (21) connecting the enclosure (45) of the selector to each of the vacuum chambers (12), these circuits being arranged radially on the enclosure (45) in such locations that the rotation of the selector cylinder (46) by a drive means (49) connected to it makes the orifice (50) in the side wall of the selector cylinder coincide in succession with the pipes (21) arranged radially on the enclosure, so as to ensure that the various vacuum chambers (12) are put in communication with the pressure-measuring system (23) in succession.

10. Detection device according to any one of Claims 1 to 9, characterised in that the low-conductivity elements (18) have a conductivity which is adjustable independently of one another.

11. Detection device according to any one of Claims 1 to 10, characterised in that the pressure-measuring means (23) is a device for measuring the total pressure in the measuring chamber.

12. Device according to any one of Claims 1 to 11, characterised in that the pressure-measuring means (23) is connected to a computation unit (25) for processing the signals transmitted by this measuring means.

13. Use of a measuring device according to any one of Claims 1, 2 and 3 for monitoring the leaks at various points of a circuit or of a system containing at least one fluid, characterised in that samples are taken in regions adjacent to various points of the circuit or of the system so as to detect traces of fluid in the atmosphere surrounding the circuit or the system.

14. Use of a measuring device according to any one of Claims 1, 2 and 3 for monitoring the leak-proofness of an exchange wall separating two fluids in a system such a heat exchanger, characterised in that a substance is introduced as a tracer into one of the fluids, or first fluid, and in that samples of a second fluid are taken at various points of the system so as to detect in these samples the possible presence of the tracer constituting the substance which is to be measured.

## Ansprüche

1. Vorrichtung zum Suchen einer Substanz, deren Anwesenheit in einer oder mehreren Kammern (12) einer Gruppe Meßkammern durch aufeinanderfolgende Druckmessung in den jeweiligen Meßkammern (12) festgestellt werden soll, in denen über nur eine Pumpanlage (14) Ultrahochvakuum erzeugt wird und die jeweils über ein Teil (11) das einen begrenzten und wahlweisen Durchlaß der Substanz zuläßt, mit einer Stelle eines Kreises oder einer Anlage, der oder die ein die Substanz bildende oder die Substanz enthaltende Medium aufnimmt, dadurch gekennzeichnet, daß die Meßkammern (12) an der einzigen Pumpanlage (14) über Elemente (18) mit niedrigem Wirkleitwert, d. h. die einen großen Widerstand gegen den Moleküldurchgang aufweisen, angeschlossen sind, und anderseits mit einem der Kammeranordnung gemeinsamen Druckmeßmittel (23) über ein Verstellglied (20) wahlweise verbunden werden, mit dem die jeweiligen Kammern (12) mit dem Druckmeßmittel (23) nacheinander in Verbindung gesetzt werden können.

2. Suchvorrichtung nach Anspruch 1, dadurch gekennzeichnet, daß das einen begrenzten Durchgang der Substanz zulassenden Teil (11) eine Wand ist, in welcher die Substanz diffundiert werden kann.

3. Suchvorrichtung nach Anspruch 1, dadurch gekennzeichnet, daß das einen begrenzten Durchgang der Substanz bietenden Teil (11) aus mindestens einem Kapillarröhrchen gebildet ist.

4. Suchvorrichtung nach Anspruch 2, wenn die zu messende Substanz Wasserstoff ist, der in einem in einer Anlage mit einem Wärmetauscher umlaufenden flüssigen Metall enthalten ist, wobei das flüssige Metall das eine Tauschmedium bildet und das andere Tauschmedium Wasserstoff enthält, dadurch gekennzeichnet, daß sie eine Anordnung Kreise zur Entnahme von flüssigem Metall und zur Trennung von Wasserstoff aufweist, wobei jeder Kreis einer an einem besonderen Ort in der Anlage liegenden Entnahmestelle zugeordnet ist und umfaßt :
— eine Flüssigmetall-Zulaufleitung (4), die das flüssige Metall bei konstantem Durchsatz aufnimmt und einen der Anordnung der Kreise gemeinsamen Sparwärmetauscher (9) durchquert,
— und eine Wasserstofftrennvorrichtung mit einer ersten Kammer (10), welche einerseits an die Flüssigmetall-Zulaufleitung (4) und anderseits an eine Rücklaufleitung (15) dieses Metalls zu dem Sparwärmetauscher (9) angeschlossen ist und eine zweite von der ersten durch eine Metallwand (11) getrennte Kammer aufweist, welche die Tiefdruck-Meßkammer (12) bildet.

5. Suchvorrichtung nach Anspruch 4, dadurch gekennzeichnet, daß die Zulaufleitungen (4) als Bypass jeweils auf einer Entnahmeleitung (2) angeordnet sind, auf welcher unterhalb der Abzweigung der Zulaufleitung (4) ein Flüssigmetall-Durchsatzregler (27) angeordnet ist.

6. Suchvorrichtung nach einem der Ansprüche 4 und 5, dadurch gekennzeichnet, daß der Sparwärmetauscher (9) aus einem die Flüssigmetall-Zulaufleitungen (4) umgebenden Hohlkörper (30) besteht, der den Flüssigmetall-Rücklaufkreis unterhalb der Rücklaufleitungen (15) der verschiedenen Entnahmekreise bildet, die alle an ihrem Ende mit dem Sparwärmetauschergehäuse in Verbindung stehen, wobei oberhalb des Sparwärmetauschers (9) das zurücklaufende Flüssigmetall innen in den Leitungen (15) der Entnahmekreise über einen Vorwärmer (16) erhitzt werden kann, und wobei die Zulaufleitungen (4) durch das aus den verschiedenen Entnahmekreise kommende Flüssigmetall erhitzt werden, dessen Mischung in dem Sparwärmetauscher erfolgt.

7. Vorrichtung nach Anspruch 6, dadurch gekennzeichnet, daß der Metall-Vorwärmer (16) in den Rücklaufleitungen (15) der Entnahmekreise aus einem einzigen Magnetkreis (38) besteht, der zur Erzeugung der Induktion für die Erhitzung des flüssigen Metalls in den Leitungen (15) von einer Wicklung (37) umgeben ist.

8. Vorrichtung nach Anspruch 5, dadurch gekennzeichnet, daß der Flüssigmetall-Durchsatzregler für die jeweiligen Kreise aus einem konischen mit seiner senkrechten Achse angeordneten Leitungsabschnitt (27) besteht, der an seinem Fußende durch die Entnahmeleitung (2) versorgt wird und an seinem Oberteil an die Flüssigmetall-Rücklaufleitung (6) angeschlossen ist, und in welchem ein Schwimmer (26) angeordnet ist, der die konische Leitung gemäß seiner senkrechten Lage in dieser Leitung mehr oder weniger verschließt, wobei die Flüssigmetall-Zulaufleitung (4) als Bypass am Fußende der konischen Leitung (27) angeordnet ist, und wobei die jeweils den jeweiligen Entnahmekreise zugeordneten Durchsatzregelvorrichtungen (27) einen gleichen Lastverlust in jedem Entnahmekreis erzeugen.

9. Vorrichtung nach einem der Ansprüche 1 bis 8, dadurch gekennzeichnet, daß die Verstellvorrichtung (20) aus einem dichten Zylindermantel (45) besteht, in welchem ein Verstellzylinder (46) mit einer Seitenöffnung (50) koaxial zum Mantel (45) angeordnet und um seine Achse in diesem Mantel drehbar montiert ist, wodurch ein mit dem Druckmeßmittel (23) verbundenes Teil (51) des Mantels der Verstellvorrichtung nacheinander mit den Leitungen (21) in Verbindung gesetzt werden kann, die den Mantel (45) der Verstellvorrichtung mit jeder Vakuumkammer (12) verbinden, wobei diese Kreise an Stellen radial an dem Mantel (45) derart angeordnet sind, daß bei Drehung des Verstellzylinders (46) durch ein mit diesem verbundenen Motormittel (49) die Öffnung (50) in der Verstellzylinderseitenwand nacheinander mit den an dem Mantel radial angeordneten Leitungen (21) zusammenfällt um die aufeinanderfolgende Herstellung der Verbindung der einzelnen Vakuumkammern (12) mit dem Druckmeßsystem (23) zu gewährleisten.

10. Vorrichtung nach einem der Ansprüche 1 bis 9, dadurch gekennzeichnet, daß die Elemente mit niedrigem Wirkleitwert (18) einen unabhängig voneinander einstellbaren Wirkleitwert haben.

11. Vorrichtung nach einem der Ansprüche 1 bis 10, dadurch gekennzeichnet, daß das Druckmeßmittel (23) eine Vorrichtung zur Messung des Gesamtdruckes in der Meßkammer ist.

12. Vorrichtung nach einem der Ansprüche 1 bis 11, dadurch gekennzeichnet, daß das Druckmeßmittel (23) an eine Recheneinheit (25) zur Bearbeitung der von diesem Meßmittel abgegebenen Signale angeschlossen ist.

13. Anwendung einer Vorrichtung nach einem der Ansprüche 1, 2 und 3, zur Leckprüfung an verschiedenen Stellen eines Kreises oder einer Anlage mit mindestens einem Medium, dadurch gekennzeichnet, daß zum Nachweis von Mediumspuren in der den Kreis oder die Anlage umgebenden Atmosphäre Entnahmen in benachbarten Zonen der verschiedenen Kreis- oder Anlagestellen vorgenommen werden.

14. Anwendung einer Vorrichtung nach einem der Ansprüche 1, 2 und 3, zur Dichtheitprüfung einer Austauschwand, die zwei Medien in einer Anlage wie ein Wärmetauscher trennt, dadurch gekennzeichnet, daß einem Medium oder dem ersten Medium eine Substanz als Indikator zugesetzt wird, und daß an verschiedenen Stellen der Anlage vom zweiten Medium Entnahmen vorgenommen werden, um in diesen Entnahmen die evtl. Anwesenheit des Indikators, der die zu messende Substanz bildet, nachzuweisen.

Fig 1

Fig 2